# EUROPÄISCHE PATENTSCHRIFT

(11) **EP 1 082 283 B1**
(45) Veröffentlichungstag und Bekanntmachung des Hinweises auf die Patenterteilung: **23.04.2003**
(21) Anmeldenummer: 99924922.0
(22) Anmeldetag: 10.05.1999
(51) Int. Cl.: C07C 29/149, C07C 31/20, C07C 51/47, C07C 67/03, C07C 67/56, C07C 69/44

(54) **VERFAHREN ZUR HERSTELLUNG VON HEXANDIOL-1,6**
METHOD FOR PRODUCING HEXANEDIOL-1,6
PROCEDE DE PREPARATION D'HEXANEDIOL-1,6

(30) Priorität: 29.05.1998 DE 19826614
(43) Veröffentlichungstag der Anmeldung: 14.03.2001
(73) Patentinhaber: BASF AKTIENGESELLSCHAFT, 67056 Ludwigshafen (DE)
(72) Erfinder: STEIN, Frank, D-67098 Bad Dürkheim (DE); KRUG, Thomas, D-67549 Worms (DE); GALL, Martin, D-67112 Mutterstadt (DE); IFFLAND, Gabriele, D-67071 Ludwigshafen (DE)
(86) Internationale Anmeldenummer: EP9903193
(87) Internationale Veröffentlichungsnummer: WO99062852

(56) Entgegenhaltungen:
- WO-A-97/31882
- WO-A-97/31883
- DE-A- 2 819 593
- DE-A- 19 514 930
- US-A- 3 551 300

## Beschreibung

Die Erfindung betrifft ein Verfahren zur Herstellung von Hexandiol-1,6 aus einem Adipinsäure und 6-Hydroxycapronsäure enthaltenden Carbonsäuregemisch, das bei der Oxidation von Cyclohexan zu Cyclohexanon/Cyclohexanol mit Sauerstoff und durch Wasserextraktion des Reaktionsgemisches erhalten wird, durch Veresterung der Säuren und Hydrierung, wobei man ein Kobalt und Phosphat als Verunreinigungen enthaltendes Carbonsäuregemisch verwendet und man dieses Gemisch zur Entfernung dieser Verunreinigungen über einen Kationenaustauscher und anschließend das Veresterungsgemisch über einen Anionenaustauscher leitet.

Aus WO 97/31882 ist ein Verfahren bekannt, Hexandiol-1,6 aus wäßrigen Lösungen von Carbonsäuren herzustellen, die bei der Oxidation von Cyclohexan zu Cyclohexanon/Cyclohexanol mit Sauerstoff und Wasserextraktion anfallen, wobei man
a) die in dem wäßrigen Dicarbonsäuregemisch enthaltenen Monound Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag in an sich bekannter Weise Hexandiol-1,6 gewinnt.

Es hat sich nun gezeigt, daß wäßrige Carbonsäuregemische, sofern sie als Verunreinigung aus der Oxidation Kobalt in Form von Co²⁺ und/oder Co³⁺-Ionen in Mengen von z.B. 20 bis 300 ppm und Phosphat in Mengen von z.B. 40 bis 1500 ppm enthalten nicht unmittelbar für das Verfahren gemäß WO 97/31882 geeignet sind. Es bestand daher die Aufgabe ein Verfahren vorzuschlagen, die genannten Verunreinigungen in einfacher und wirtschaftlicher Weise aus der Einsatzlösung zu entfernen.

Diese Aufgabe wurde erfindungsgemäß gelöst mit einem Verfahren zur Herstellung von Hexandiol-1,6 aus einem Adipinsäure, 6-Hydroxycapronsäure und geringe Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren und Hydrierung, wobei man
a) die in dem wäßrigen Dicarbonsäuregemisch enthaltenen Monound Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag in an sich bekannter Weise Hexandiol-1,6 gewinnt, wobei man erfindungsgemäß ein wäßriges Dicarbonsäuregemisch verwendet, das mehr als 20 ppm Kobalt und mehr als 40 ppm Phosphor in Form von Phosphat enthält und daß man dieses wäßrige Dicarbonsäuregemisch über einen Kationenaustauscher und nach der Veresterung gemäß Stufe (a) über einen Anionenaustauscher leitet.

Obwohl die Verwendung von Ionenaustauschern zur Entfernung von Kationen bzw. Anionen allgemeiner Stand der Technik ist, war es überraschend, daß bei dem erfindungsgemäßen Verfahren keine unerwünschte Nebenreaktionen bei der zwischen dem Kationenaustauscher und dem Anionenaustauscher befindlichen Entwässerung eintraten. Im sauren Kationenaustauscher wird nämlich das Phosphat in Phosphorsäure umgewandelt, so daß damit gerechnet werden mußte, daß bei der destillativen Entwässerung der mit dem Kationenaustauscher behandelten Carbonsäurelösung sauer katalysierte Reaktionen der 6-Hydroxycapronsäure auftreten würden, wie Eliminierung der Hydroxylgruppe oder Polyesterbildung. Beides ist jedoch nicht der Fall. Die Bildung der Phosphorsäure hat sogar den Vorteil, daß sie als Veresterungskatalysator dienen kann und die Menge der zuzusetzenden Schwefelsäure als Veresterungskatalysator verringert werden kann.

Das erfindungsgemäße Verfahren ist abgesehen von der Behandlung des wäßrigen Carbonsäuregemischs mit dem Kationenaustauscher und des Veresterungsreaktionsgemisches mit dem Anionenaustauscher in allen Einzelheiten in WO 97/31882 beschrieben, so daß auf diese Schrift ausdrücklich Bezug genommen wird. Sämtliche dort gemachten Angaben sollen auch hier ohne irgendwelche Beschränkungen gelten.

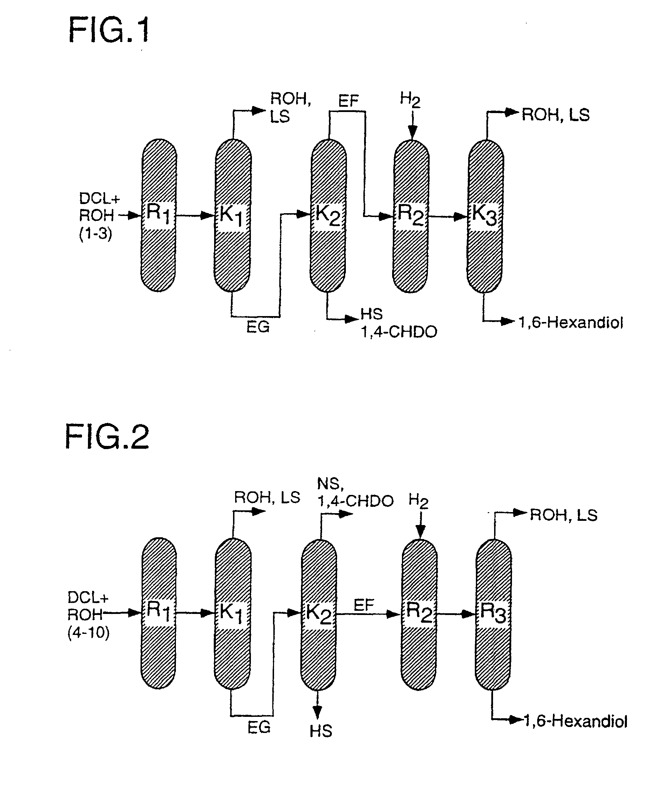

Das dort beschriebene Verfahren wird mit seinen Varianten A (Fig. 1) und Variante B (Fig. 2) hier nochmals erläutert (wobei die Begriffe über Kopf bzw. als Sumpf jeweils den Abzug oberhalb bzw. unterhalb des Zulaufs bedeuten), um den Ort der Behandlung mit dem Kationenaustauscher bzw. Anionenaustauscher besser zeigen zu können:
Variante A
   Wie in Fig. 1 dargestellt, wird die Dicarbonsäurelösung (DCL), gegebenenfalls nach Entwässerung, zusammen mit einem C₁- bis C₃-Alkohol, vorzugsweise Methanol, in den Veresterungsreaktor R₁ eingespeist, wo die Carbonsäuren verestert werden. Das erhaltene Veresterungsgemisch gelangt dann in Kolonne K₁, in der der überschüssige Alkohol (ROH), Wasser und Leichtsieder (LS) über Kopf abdestilliert und das Estergemisch (EG) als Sumpf abgezogen und in die Fraktionierkolonne K₂ eingespeist wird. In dieser Kolonne wird das Gemisch in einer im wesentlichen von 1,4-Cyclohexandiolen freie Esterfraktion (EF) und eine Sumpffraktion, bestehend aus Hochsiedern (HS) und 1,4-Cyclohexandiolen (1,4-CHDO), fraktioniert. Die Esterfraktion (EF) wird dann in dem Hydrierreaktor R₂ katalytisch hydriert und das Hydriergemisch in der Destillationskolonne K₃ in Alkohol (ROH), Leichtsieder (LS) und reines 1,6-Hexandiol aufgetrennt.
Variante B
   Verwendet man zur Veresterung Alkohole mit 4 und mehr Kohlenstoffatomen, insbesondere n- oder i-Butanol, unterscheidet sich das Verfahren gemäß Fig. 2 nur insofern, als in der Fraktionskolonne K₂ das Estergemisch (EG) in ein Kopfprodukt von Niedrigsiedern (NS), die die 1,4-Cyclohexandiole (1,4-CHDO) enthalten, und eine im wesentlichen von 1,4-Cyclohexandiol freie Esterfraktion (EF) aufgetrennt wird, die man als Seitenfraktion oder als die Esterfraktion enthaltenden Sumpf gewinnt und in die Hydrierstufe (R₂) einspeist.

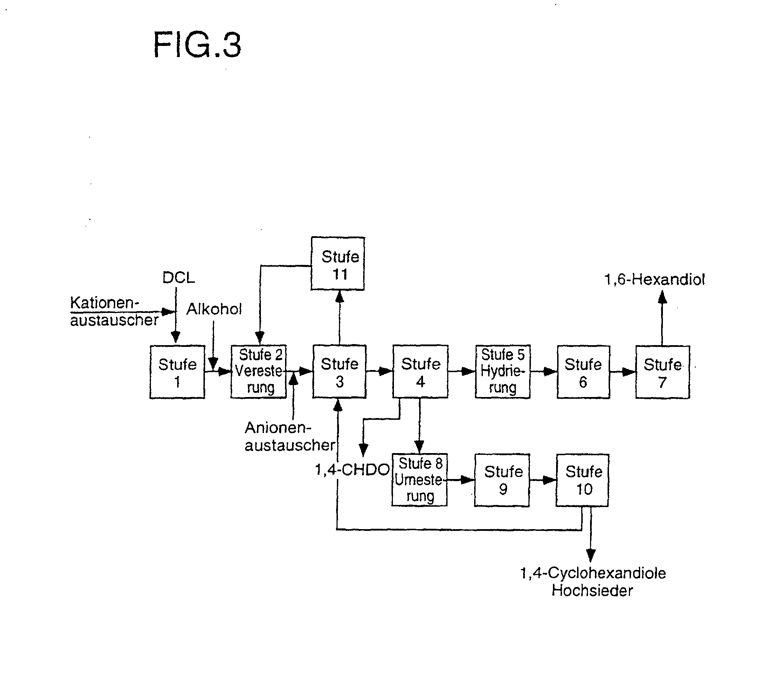

Im folgenden wird das Verfahren noch näher erläutert. Dabei sind gemäß Fig. 3 die einzelnen Verfahrensschritte in weitere Stufen aufgeschlüsselt, wobei die Stufen 0, 2, 2a, 3, 4, 5, 6, 7 für das Verfahren essentiell sind und die Stufen 3 und 4 sowie 6 und 7 auch zusammengefaßt werden können. Die Stufen 8, 9, 10 und 11 sind fakultativ, aber zur Erhöhung der Wirtschaftlichkeit des Verfahrens gegebenenfalls sinnvoll.

Die weiter unten eingehend beschriebene Behandlung mit dem Kationenaustauscher findet vor der Stufe 1 als Stufe 0 statt.

Die Dicarbonsäurelösung (DCL) ist im allgemeinen eine wäßrige Lösung mit einem Wasseranteil von 20 bis 80 %. Da eine Veresterungsreaktion eine Gleichgewichtsreaktion darstellt, ist es meist sinnvoll, insbesondere bei Veresterung mit z.B. Methanol, vorhandenes Wasser vor der Reaktion zu entfernen, vor allem, wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders bevorzugt 30 bis 200°C und einem Druck von bis 1500 mbar, bevorzugt 5 bis 1100 mbar, besonders bevorzugt 20 bis 1000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, daß das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.% betragen.

Die Abtrennung des Wassers kann so erfolgen, daß das Wasser überwiegend säurefrei erhalten wird, oder man kann die in der DCL enthaltenen niederen Monocarbonsäuren - im wesentlichen Ameisensäure - zum größten Teil mit dem Wasser abdestillieren, damit diese in der Veresterung keinen Veresterungsalkohol binden.

Dem Carbonsäurestrom aus der Stufe 1 wird ein Alkohol mit 1 bis 10 C-Atomen zugemischt, gemäß Variante A Alkohole mit 1 bis 3 Kohlenstoffatomen, d.s. Methanol, Ethanol, Propanol oder iso-Propanol, bevorzugt Methanol, gemäß Variante B Alkohole mit 4 bis 10, insbesondere 4 bis 8 Kohlenstoffatomen und besonders bevorzugt n-Butanol, iso-Butanol, n-Pentanol und i-Pentanol.

Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt bei 70 bis 300°C, besonders bevorzugt bei 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen; bevorzugt wird aber zur Erhöhung der Reaktionsgeschwindigkeit ein Katalysator zugesetzt. Dabei kann es sich um einen homogen gelösten oder um einen festen Katalysator handeln. Als homogene Katalysatoren seien beispielhaft Schwefelsäure, Phosphorsäure, Salzsäure, Sulfonsäuren, wie p-Toluolsulfonsäure, Heteropolysäuren wie Wolframatophosphorsäure oder Lewissäuren wie Aluminium-, Vanadium-, Titan-, Bor-Verbindungen genannt. Bevorzugt sind Mineralsäuren, insbesondere Schwefelsäure. Das Gew.-Verhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂ oder Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt, abzüglich der gegebenenfalls zugesetzten Säure als Katalysator, 0,01 bis 50, bevorzugt 0,1 bis 10. Dabei liegen nicht alle im System vorhandenen Carboxylgruppen als Ester des eingesetzten Alkohols vor, sondern ein Teil kann in Form von dimeren oder oligomeren Estern, z.B. mit dem OH-Ende der Hydroxycapronsäure vorliegen.

Die weiter unten eingehend beschriebene Behandlung mit dem Anionenaustauscher findet zwischen Stufe 2 und Stufe 3 als Stufe 2a statt.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150°C, bevorzugt 15 und 90°C, und insbesondere 25 und 75°C der überschüssige Veresterungsalkohol ROH, Wasser sowie z.B. entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren, wie Adipinsäure und Glutarsäure, Hydroxycarbonsäuren, wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 10 Gew.-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf im allgemeinen zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1000 mbar, bevorzugt 5 bis 500 mbar, besonders bevorzugt 10 bis 200 mbar, betrieben.

Nach Variante A, d.h. der Veresterung mit C₁- bis C₃-Alkoholen, insbesondere Methanol, wird nun der Strom aus Stufe 3 in eine zu hydrierende Kopffraktion und eine die 1,4-Cyclohexandiole enthaltende Sumpffraktion getrennt.

Die Kopffraktion besteht überwiegend aus Restwasser und Restalkohol ROH, Estern des Alkohols ROH mit Monocarbonsäuren, überwiegend C₃- bis C₆-Monocarbonsäuren, Estern mit Hydroxycarbonsäuren, wie 6-Hydroxycapronsäure, 5-Hydroxyvaleriansäure, sowie vor allem den Diestern mit Dicarbonsäuren, wie Adipinsäure, Glutarsäure und Bernsteinsäure, ferner 1,2-Cyclohexandiolen, Caprolacton und Valerolacton.

Die genannten Komponenten können zusammen über Kopf abgetrennt und in die Hydrierung (Stufe 5) eingeschleust werden oder in einer weiteren bevorzugten Ausführungsform in der Kolonne in einen Kopfstrom, der überwiegend Restwasser und Restalkohol sowie die oben erwähnten Ester der C₃- bis C₅-Carbonsäuren enthält und einen Seitenstrom, der überwiegend die oben erwähnten Ester der C₆-Carbonsäuren und Dicarbonsäuren enthält, die dann in die Hydrierung gelangen, aufgetrennt werden.

Die schwersiedenden Komponenten des Stromes aus Stufe 4, überwiegend bestehend aus 1,4-Cyclohexandiolen oder deren Ester, dimeren oder oligomeren Estern sowie nicht näher definierten z.T. polymeren Bestandteilen der DCL, werden über den Abtriebsteil der Kolonne abgetrennt. Diese können zusammen anfallen oder so, daß über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sog. Umesterung in die Stufe 8 gelangen.

Nach Variante B, d.h. der Veresterung mit C₄- bis C₁₀-Alkoholen, insbesondere n- oder i-Butanol, kann der Strom aus Stufe 3 in der Stufe 4 in eine die 1,4-Cyclohexandiole enthaltene Kopffraktion, einen vorwiegend die C₆-Ester enthaltenden Seitenstrom, der in die Hydrierung gelangt und Hochsieder enthaltenden Sumpfstrom, der gegebenenfalls in die Stufe 8 gelangen kann, aufgetrennt werden.

Die Kopffraktion besteht überwiegend aus Restalkohol ROH, C₁bis C₃-Monoestern des Alkohols ROH, Valerolacton und 1,2- und 1,4-Cyclohexandiolen.

Der Seitenstrom enthält überwiegend Diester von Bernsteinsäure, Glutarsäure und Adipinsäure sowie Monoester der 5-Hydroxyvaleriansäure und 6-Hydroxycapronsäure. Dieser Seitenstrom kann entweder oberhalb oder auch unterhalb der Zulaufstelle der Kolonne entnommen werden und in die Hydrierung (Stufe 5) eingeschleust werden.

Der Sumpfstrom mit oligomeren Estern und sonstigen Hochsiedern kann analog der Variante A entweder verbrannt oder vorteilhaft in die Stufe 8 gelangen.

Nach einer weiteren Ausführungsform werden in der Stufe 4 die C₆-Ester zusammen mit entweder dem Sumpfstrom abgetrennt und dann, in einer weiteren Kolonne, entweder als Sumpfprodukt von der bereits beschriebenen Kopffraktion, die überwiegend aus Restalkohol ROH, C₁- bis C₃-Monoestern des Alkohols ROH, Valerolacton und 1,2- und 1,4-Cyclohexandiolen besteht, oder als Kopfstrom von den Hochsiedern abgetrennt.

Die von 1,4-Cyclohexandiole freie oder praktisch freie Fraktion der Stufe 4, entweder der Gesamtstrom oder der hauptsächlich Ester der C₆-Säuren enthaltende Seitenstrom, wird in die Hydrierstufe 5 geleitet.

Die Stufen 3 und 4 können, insbesondere wenn nur kleinere Mengen verarbeitet werden, zusammengefaßt werden. Dazu kann beispielsweise in einer absatzweise durchgeführten fraktionierten Destillation der C₆-Esterstrom gewonnen werden, wiederum ohne daß 1,4-Cyclohexandiol in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind z.B. in Houben-Weyl, Methoden der Organischen Chemie, Band IV/1c, Georg Thieme Verlag Stuttgart, 1980, S. 45-67) und Beispiele für heterogene Katalysatoren sind z.B. in Houben-Weyl, Methoden der Organischen Chemie, Band IV/1c, S. 16 bis 26 beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, ZnO₂, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≧ 0, d > 0, a > b/2, b > a/4, a > c und a > d gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so daß man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m²/g.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im allgemeinen Temperaturen von 150 bis 300°C bei Drücken von 1 bis 100 bar, bevorzugt 15 bis 70 bar angewandt. Dabei wird zweckmäßig mindestens so viel Wasserstoff als Hydriermittel und Trägergas verwendet, daß Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinandergeschalteten Reaktoren durchgeführt werden. Die Hydrierung in Flüssigphase über ein Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im wesentlichen aus 1,6-Hexandiol und dem Alkohol ROH. Weitere Bestandteile sind, vor allem falls der gesamte leichtsiedende Strom der Stufe 4 gemäß Variante A eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Dieser Hydrieraustrag wird in der Stufe 6, die z.B. ein Membransystem oder bevorzugt eine Destillationskolonne ist, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1500 mbar, bevorzugt 30 bis 1200 mbar, besonders bevorzugt 50 bis 1000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, gegebenenfalls die 1,2-Cyclohexandiole, sowie weitere eventuell vorhandene Leichtsieder, über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1000 mbar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 6 und 7 auch in einer diskontinuierlichen fraktionierten Destillation zusammengefaßt werden.

Um die Hexandiol-Herstellung möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH, beispielsweise Methanol enthaltende Strom aus Stufe 3 und/oder 6 in der Stufe 11 aufgearbeitet werden. Zu diesem Zweck wird vorteilhaft eine Kolonne verwendet, in der Komponenten, die leichter sieden als der Alkohol ROH über Kopf, Wasser und Komponenten, die höher sieden als der Alkohol ROH, über Sumpf vom Alkohol ROH, der in einem Seitenstrom gewonnen wird, abgetrennt werden. Die Kolonne wird zweckmäßig bei 500 bis 5000 mbar, bevorzugt bei 800 bis 3000 mbar, betrieben.

Nach einer weiteren bevorzugten Ausführungsform des Verfahrens wird der hochsiedende Strom aus Stufe 4 (gemäß Variante A) zur Erhöhung der Gesamtausbeute an 1,6-Hexandiol bezogen auf eingesetzte Adipinsäure und 6-Hydroxycapronsäure in der eingesetzten DCL, verwendet. Dazu wird in der Stufe 8 der Anteil an dimeren und oligomeren Estern der Adipinsäure bzw. Hydroxycapronsäure mit weiteren Mengen des Alkohols ROH in Gegenwart eines Katalysators umgesetzt. Das Gew.-Verhältnis von Alkohol ROH und dem Sumpfstrom aus Stufe 4 beträgt zwischen 0,1 bis 20, bevorzugt 0,5 bis 10, besonders bevorzugt 1 bis 5. Als Katalysatoren eignen sich prinzipiell die bereits für die Veresterung in Stufe 2 beschriebenen. Bevorzugt werden jedoch Lewissäuren eingesetzt. Beispiele hierzu sind Verbindungen oder Komplexe des Aluminiums, Zinns, Antimons, Zirkons oder Titans, wie Zirkoniumacetylacetonat oder Tetraalkyltitanate, z.B. Tetraisopropyltitanat, die in Konzentrationen von 1 bis 10000 ppm, bevorzugt 50 bis 6000 ppm, besonders bevorzugt 100 bis 4000 ppm, bezogen auf das Umesterungsgemisch, angewandt werden. Besonders bevorzugt hierbei sind Titanverbindungen.

Die Umesterung kann absatzweise oder kontinuierlich, in einem Reaktor oder mehreren Reaktoren, in Reihe geschaltenen Rührkesseln oder Rohrreaktoren bei Temperaturen zwischen 100 und 300°C, bevorzugt 120 bis 270°C, besonders bevorzugt 140 bis 240°C und den sich dabei einstellenden Eigendrücken, durchgeführt werden. Die benötigten Verweilzeiten liegen bei 0,5 bis 10 Stunden, bevorzugt bei 1 bis 4 Stunden.

Dieser Strom aus der Stufe 8 läßt sich im Falle der Veresterung mit Methanol z.B. wieder in die Stufe 3 einschleusen. Zur Vermeidung von Aufpegelungen, vor allem von 1,4-Cyclohexandiolen, muß dann absatzweise oder kontinuierlich ein Teilstrom der Hochsieder aus Stufe 4 ausgeschleust werden. Eine andere Möglichkeit ist, den Strom aus Stufe 8 nicht in Stufe 3 zurückzuführen, sondern ihn, analog zur Stufe 3, in einer Stufe 9 in vorwiegend Alkohol ROH, der dann wieder in die Stufe 2, 8 oder 11 gelangen kann, und einen Strom, der die Ester enthält, aufzutrennen.

Dieser Esterstrom kann prinzipiell (mit der Maßgabe der Vermeidung von Aufpegelungen der 1,4-Cyclohexandiole) in die Stufe 4 zurückgeführt werden oder wird bevorzugt in eine weitere Stufe 10, in die Ester der C₆-Säuren und, mengenmäßig eher unbedeutend, in die Ester der C₅-Säuren einerseits, die entweder in die Stufe 4 oder direkt in die Stufe 5 eingeschleust werden können und Hochsieder andererseits, die die 1,4-Cyclohexandiole enthalten, aufgetrennt, worauf die Hochsieder ausgeschleust werden.

Auf diese Weise lassen sich Ausbeuten an 1,6-Hexandiol von über 95 %, bei Reinheiten von über 99 % erzielen.

Im einzelnen geht man bei der Behandlung des Edukts mit dem Kationenaustauscher so vor, daß man vorzugsweise ein Festbettionenaustauscher verwendet, der ein stark saurer, gelförmiger Kationenaustauscher oder bevorzugt ein stark saures makroporöses hochvernetztes Harz sein kann.

Die Behandlung mit dem Kationenaustauscher führt man bevorzugt in zwei wechselweise betriebenen Festbetten, die auch hintereinander geschaltet werden können durch, wobei zunächst nur das erste Bett mit Kobalt beladen wird. Spätestens wenn eine Konzentration von 10 ppm Kobalt im Austrag erreicht ist, leitet man die wäßrige Carbonsäurelösung zusätzlich über das zweite Bett, bis das erste Bett erschöpft ist, d.h. der Austrag des ersten Bettes z.B. mehr als 90 % der Zulaufkonzentration erreicht hat oder ein vollständiger Durchbruch des nicht mehr absorbierten Kobalts erfolgt. Der Feedstrom wird dann direkt auf das zweite Festbett geleitet. Das ursprünglich erste Bett wird dann regeneriert, vorzugsweise nachdem vorher noch in dem Ionenaustauscher zurückgehaltenes Produkt mit Wasser ausgewaschen und das Eluat in den Eduktstrom zurückgeführt worden ist und übernimmt nach der Regeneration die Funktion des nachgeschalteten Bettes.

Mit den erfindungsgemäß zu verwendenden Kationenaustauschern lassen sich die Kobaltgehalte auf Werte von 1 ppm und weniger reduzieren.

Als Kationenaustauscher werden an sich bekannte Austauscherharze, wie z.B. schwach saure, stark saure oder Chelatbildner verwendet, wie sie z.B. in "Ionenaustauscher, Dr. U. Dorfner, 1970, Walter de Gruyter & Co, Berlin" beschrieben sind. Besonders bevorzugt sind stark saure Kationenaustauscher.

Insbesondere kommen in einem Polymergerüst gebundene Sulfonsäuregruppen oder Carbonsäuregruppen enthaltende Kationenaustauscher der Zusammensetzung Polystyrol vernetzt mit Divinylbenzol in Betracht. Je nach Gehalt an Divinylbenzol handelt es sich um gelförmige (Divinylbenzolgehalt ∼ 4 %) oder makroporöse (Divinylbenzolgehalt ∼ 12 - 20 %) Ionenaustauscherharze.

Es sind auch Kationenaustauscher auf Basis Acrylsäure oder Methacrylsäure vernetzt mit Divinylbenzol oder Harze hergestellt durch Kondensation von Formaldehyd und Phenol einsetzbar.

Im einzelnen sind z.B. Lewatit® CNP 80, Lewatit SP 112, Lewatit K 2621, Lewatit 10 P 80 der Firma Bayer und Amberlite® 252C, Amberlite 1200 und Duolite® AR C9652 oder Amberlyst® WET der Fa. Rohm & Haas zu nennen, wie sie in den Firmenschriften dieser Firmen beschrieben sind.

Zur Entfernung der unerwünschten Anionen, das ist vor allem Phosphat und auch Sulfat (aus Schwefelsäure als Veresterungskatalysator), verwendet man wie für den Kationenaustauscher beschrieben bevorzugt ebenfalls 2 wechselweise betriebene Festbetten, die auch hintereinandergeschaltet sein können. Dazu wird das Veresterungsgemisch solange über das erste Bett geleitet, bis der Phosphatgehalt im Austrag z.B. 10 ppm erreicht. Dies kann über ein Ansteigen der Leitfähigkeit und einen Abfall des pH-Werts festgestellt werden. Nach Erreichen dieser Grenze schaltet man ein zweites Anionenaustauscherbett dahinter, bis der erste Ionenaustauscher erschöpft ist, d.h. bis die Konzentration an Phosphat im Austrag z.B. 50 % des Zulaufwertes erreicht oder ein völliger Durchbruch erfolgt. Dies ist in der Regel bei einem Absinken des pH-Werts unter 4 der Fall. Dann wird das erste Bett abgeschaltet und regeneriert. Vor der Regenerierung wird zweckmäßigerweise das im Ionenaustauscher zurückgehaltene Estergemisch ausgewaschen. Dies geschieht im allgemeinen durch Spülen mit Stickstoff, Methanol und erneut Stickstoff und Rückführung des Eluats in die Veresterungsstufe. Das regenerierte erste Bett übernimmt dann die Funktion des nachgeschalteten Bettes.

Als Anionenaustauscher kommen ebenfalls an sich bekannte, stark basische, schwach basische, mittel basische gelförmige oder makroporöse Ionenaustauscher in Betracht, wie sie in "Ionenaustauscher, Dr. U. Dorfner, 1970, Walter de Gruyter & Co, Berlin" beschrieben sind. Dies sind in der Regel schwach basische makroporöse Ionenaustauscher, vorzugsweise mit besonders geringer Basizität. Dies sind z.B. Anionenaustauscher der Struktur Polystyrolharze mit Divinylbenzol vernetzt mit tertiären Aminogruppen als funktionelle Gruppen. Auch Ionenaustauscher mit stark basischen Gruppen (quartäre Amine) oder mit mittelbasischen Gruppen können erfindungsgemäß verwendet werden. Auch Anionenaustauscher auf Basis Acrylsäure oder Methacrylsäure vernetzt mit Divinylbenzol oder Harze hergestellt durch Kondensation von Formaldehyd und Phenol kommen in Betracht.

Im einzelnen kommen z.B. Amberlite® IRA 92, IRA 96, Lewatit® MP 64, MP 500, Amberjet 4200 oder Lewatit® MP 62 der Firmen Bayer AG und Rohm & Haas in Betracht (vgl. Produktblätter dieser Firmen).

Mit der erfindungsgemäßen Behandlung des Edukts bzw. des Veresterungsgemisches läßt sich das Verfahren der WO 97/31882 störungsfrei und kontinuierlich auch mit einer Kobalt und Phosphat enthaltenden Ausgangslösung betreiben. Durch die Elimination der Sulfationen ergeben sich ferner Vorteile, da bei der Neutralisation die Bildung von z.B. K₂SO₄ vermieden werden kann, dessen Anwesenheit im zu verbrennenden Abfallstrom ein besonderes, sehr kostenintensives Design des Verbrennungsofens erforderlich macht. Kaliumsulfat bildet mit den üblichen Ofenausmauerungen eutektische Gemische, so daß durch die Absenkung der Schmelztemperatur der Auskleidung die Standzeit des Boilers stark reduziert wird.

Das Verfahren wird anhand des nachfolgenden Beispiels näher erläutert aber in keiner Weise eingeschränkt.

### Beispiel (Variante A)

### Stufe 0 (Kationenaustausch)

4,7 kg/h Dicarbonsäurelösung wurden bei einer Leerrohrgeschwindigkeit von 14 m/h über eine mit Amberlyst® 15 WET gefüllte auf 60°C gehaltene Säule (Durchmesser 20 mm, Harzbetthöhe 2000 mm) gepumpt und dem Ablauf wurden Proben zur Bestimmung der Kobalt- und Eisenkonzentrationen entnommen. Die Zulauflösung hatte einen Co-Gehalt von 115 ppm (und einen Eisengehalt von 9 ppm). Beide Kationen wurden auf einen Gehalt von < 1 ppm abgereichert. Die erreichte Gesamtarbeitskapazität bis zum Durchbruch betrug 0,823 mol/l, das entspricht 1,703 val/1.

Bei einer Leerrohrgeschwindigkeit von ca. 33 m/h wurde eine Gesamtarbeitskapazität von 0,70 mol/l, entsprechend 1,45 val/l erreicht. Auch hier erzielte man eine Abreicherung auf kleiner 1 ppm Kobalt und Eisen.

### Stufe 1 (Entwässerung):

0,1 kg/h Dicarbonsäurelösung (bestehend im wesentlichen aus Adipinsäure, 6-Hydroxycapronsäure, 1,4-Cyclohexandiolen, Glutarsäure, 5-Hydroxyvaleriansäure, Ameisensäure, Wasser und mit einem Restkobaltgehalt von < 1 ppm) wurden kontinuierlich in eine Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden), mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (5,5 kg) betrug der Wassergehalt ca. 0,4 %.

### Stufe 2 (Veresterung):

5,5 kg/h des Sumpfstroms aus Stufe 1 wurden mit 8,3 kg/h Methanol und 14 g/h Schwefelsäure kontinuierlich in einem Rohrreaktor (1 0,7 m, 0̸ 1,8 cm, Verweilzeit 2,7 h) umgesetzt. Die Säurezahl des Austrags abzüglich Schwefelsäure betrug ca. 10 mg KOH/g.

### Stufe 2a (Anionenaustausch)

550 g/h Veresterungsaustrag wurden mit einer Leerrohrgeschwindigkeit von ca. 2 m/h über eine auf 35°C gehaltene und mit Lewatit® MP 62 (Bayer) gefüllte Säule gepumpt. Der ablaufenden Lösung wurden Proben entnommen und der Gehalt an Schwefel und Phosphor bestimmt. Beide Anionen konnten auf < 1 ppm abgereichert werden bei Konzentrationen der Zulauflösungen von 1050 ppm Sulfat und 950 ppm Phosphat. Die erreichte Gesamtkapazität betrug 1,2 mol/l, entsprechend 1,84 val/l (Basis SO₄²⁻ und H₂PO₄⁻). Im Verlauf eines Dauerversuches von 21 Zyklen konnte kein Abfall der Arbeitskapazität beobachtet werden. Auch mit einer Leerrohrgeschwindigkeit von 7 m/h wurde eine Abreicherung auf unter 1 ppm erzielt.

### Stufe 3 (Entfernen überschüssigen Alkohols und von Wasser):

In einer 20 cm Füllkörperkolonne wurde der Veresterungsstrom aus Stufe 2, der < 1 ppm Phosphat und < 1 ppm Sulfat enthielt, destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 4 (Fraktionierung; 1,4-Cyclohexandiolabtrennung):

In einer 50 cm Füllkörperkolonne wurde der Sumpfstrom aus Stufe 3 fraktioniert destilliert (1 mbar, 70-90°C Kopftemperatur, bis 180°C Sumpftemperatur). Der Sumpf (1,9 kg) enthielt praktisch alle 1,4-Cyclohexandiole.

Als Leichtsieder wurden 0,6 kg abdestilliert (1,2-Cyclohexandiole, Valerolacton, 5-Hydroxyvaleriansäuremethylester, Glutarsäuredimethylester, Bernsteinsäuredimethylester u.a.). Als überwiegend Adipinsäuredimethylester und 6-Hydroxycapronsäuremethylester enthaltende Fraktion wurden 4,3 kg erhalten.

Der die Esterfraktion darstellende Kopfstrom wird in Hydrierstufe 5 geleitet.

### Stufe 5 (Hydrierung):

4,3 kg der C₆-Esterfraktion aus Stufe 4 wurden kontinuierlich in einem 25-ml-Reaktor an einem Katalysator hydriert (Katalysator, 70 Gew.-% CuO, 25 Gew.-% ZnO, 5 Gew.-% Al₂O₃), der zuvor im Wasserstoffstrom bei 180°C aktiviert worden war. Der Zulauf betrug 20 g/h, der Druck 220 bar und die Temperatur 220°C). Der Ester-Umsatz betrug 99,5 %, die 1,6-Hexandiolselektivität betrug über 99 %.

Alternativ wurde die Esterfraktion in einer zweistufigen Reaktorkaskade (1. Reaktor 2,5 1 Katalysator, Rieselfahrweise, 250 bar, Produktrückführung : Zulauf = 10 : 1, 220 - 230°C; 2. Reaktor 0,5 1 Katalysator, Rieselfahrweise gerader Durchgang, 260 bar, 220°C) kontinuierlich hydriert. Als Katalysator wurde ein zuvor bei 180°C aktivierter Katalysator aus CuO (60 %), Al₂O₃ (30 %) und Mn₂O₃ (10 %) eingesetzt. Die Zulaufmenge betrug 1 kg/h. Bei 99,5 % Umsatz betrug die Hexandiol-Selektivität über 99 %.

### Stufe 6 und 7:

4,0 kg des Hydrieraustrags aus Stufe 5 wurden fraktioniert destilliert (Destillationsblase mit aufgesetzter 70 cm Füllkörperkolonne, Rücklaufverhältnis 2) Bei 1013 mbar wurde 1 kg Methanol abdestilliert. Nach Anlegen von Vakuum (20 mbar) destillierten überwiegend die 1,2-Cyclohexandiole und 1,5-pentandiol ab. Danach (Sdp. 146°C) destillierte 1,6-Hexandiol mit einer Reinheit von 99,8 % ab. (Restgehalt überwiegend 1,5-Pentandiol.)

Die Ausbeute war die gleiche wie bei Verwendung eines DCL-Eduktes, das verfahrensbedingt bereits weniger als 20 ppm Kobalt und weniger als 40 ppm Phosphat enthielt und deshalb nicht mit dem Kationen- und Anionenaustauscher behandelt wurde.

## Patentansprüche

1. Verfahren zur Herstellung von Hexandiol-1,6 aus einem Adipinsäure, 6-Hydroxycapronsäure und geringe Mengen 1,4-Cyclohexandiole enthaltenden Carbonsäuregemisch, das als Nebenprodukt bei der Oxidation von Cyclohexan zu Cyclohexanon/Cyclohexanol mit Sauerstoff oder Sauerstoff enthaltenden Gasen und durch Wasserextraktion des Reaktionsgemischs erhalten wird, durch Veresterung der Säuren und Hydrierung, wobei man
a) die in dem wäßrigen Dicarbonsäuregemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) die im wesentlichen von 1,4-Cyclohexandiolen freie Esterfraktion katalytisch hydriert und
e) in einer Reindestillationsstufe aus dem Hydrieraustrag in an sich bekannter Weise Hexandiol-1,6 gewinnt,
**dadurch gekennzeichnet, daß** man ein wäßriges Dicarbonsäuregemisch verwendet, das mehr als 20 ppm Kobalt und mehr als 40 ppm Phosphor in Form von Phosphat enthält und daß man dieses wäßrige Dicarbonsäuregemisch über einen Kationenaustauscher und nach der Veresterung gemäß Stufe (a) über einen Anionenaustauscher leitet.

2. Verfahren nach Anspruch 1, **dadurch gekennzeichnet, daß** man die Vorbehandlung mit Festbettionenaustauschern durchführt.

3. Verfahren nach Anspruch 1, **dadurch gekennzeichnet, daß** man die Vorbehandlung mit zwei wechselweise betriebenen Festbetten, die auch hintereinander geschaltet werden können, durchführt, bis der Kobaltgehalt des Auslaufs des ersten Bettes mehr als 90 % der Zulaufkonzentration beträgt, darauf Abschaltung und Regenerierung des ersten Bettes, das dann die Funktion des nachgeschalteten Bettes übernimmt.

4. Verfahren nach Anspruch 1, **dadurch gekennzeichnet, daß** man die Vorbehandlung mit jeweils 2 hintereinander geschalteten Anionenaustauscherbetten durchführt, bis der Phosphatgehalt des Auslaufs des ersten Bettes mehr als 50 % des Zulaufwertes beträgt, darauf Abschaltung und Regenerierung des ersten Bettes, das dann die Funktion des nachgeschalteten Bettes übernimmt.

5. Verfahren nach Anspruch 1, **dadurch gekennzeichnet, daß** man den Kationenaustauscher vor der Regenerierung mit Wasser produktfrei wäscht und das Waschwasser der Ausgangsdicarbonsäurelösung zumischt.

6. Verfahren nach Anspruch 1, **dadurch gekennzeichnet, daß** man den Anionenaustauscher vor der Regenerierung mit Methanol wäscht und die Waschlösung in die Veresterung zurückführt.

## Claims

1. A process for preparing hexanediol-1,6 from a carboxylic acid mixture comprising adipic acid, 6-hydroxycaproic acid and small amounts of 1,4-cyclohexanediols, said mixture being by-producted in the oxidation of cyclohexane to cyclohexanone/cyclohexanol with oxygen or oxygen-containing gases and extracted from the reaction mixture using water, by esterifying the acids and hydrogenating by
a) reacting the mono- and dicarboxylic acids contained in the aqueous dicarboxylic acid mixture with a low molecular weight alcohol to give the corresponding carboxylic esters,
b) freeing the esterification mixture obtained in a first distillation stage of excess alcohol and low boilers,
c) carrying out a separation of the bottom product in a second distillation stage into an ester fraction substantially free of 1,4-cyclohexanediols and a fraction comprising at least the larger part of the 1,4-cyclohexanediols,
d) catalytically hydrogenating the ester fraction substantially free of 1,4-cyclohexanediols and
e) obtaining hexanediol-1,6 in a manner known per se from the hydrogenation effluent in a purification by distillation stage,
which comprises using an aqueous dicarboxylic acid mixture comprising more than 20 ppm of cobalt and more than 40 ppm of phosphorus in the form of phosphate and passing this aqueous dicarboxylic acid mixture over a cation exchanger and, after the esterification of stage (a), over an anion exchanger.

2. A process as claimed in claim 1, wherein the pretreatment is carried out using fixed bed ion exchangers.

3. A process as claimed in claim 1, wherein the pretreatment is carried out using two fixed beds operated in rotation which may also be connected in series until the cobalt content of the effluent of the first bed is more than 90% of the feed concentration, followed by shutdown and regeneration of the first bed which then takes over the function of the downstream bed.

4. A process as claimed in claim 1, wherein the pretreatment is carried out in each case using two anion exchanger beds connected in series until the phosphate content of the effluent of the first bed is more than 50% of the feed value, followed by shutdown and regeneration of the first bed which then takes over the function of the downstream bed.

5. A process as claimed in claim 1, wherein the cation exchanger is washed with water to free it of product before regeneration and the washing water is admixed with the starting dicarboxylic acid solution.

6. A process as claimed in claim 1, wherein the anion exchanger is washed with methanol before regeneration and the washing solution is recycled into the esterification.

## Revendications

1. Procédé pour préparer l'hexanediol-1,6 à partir d'un mélange d'acides carboxyliques contenant de l'acide adipique, de l'acide 6-hydroxycaproïque et de faibles quantités de 1,4-cyclohexanediols, que l'on obtient en tant que produit secondaire lors de l'oxydation du cyclohexane en cyclohexanone/cyclohexanol avec de l'oxygène ou avec des gaz contenant de l'oxygène et par extraction à l'eau du mélange réactionnel, au moyen de l'estérification des acides et d'une hydrogénation, dans lequel
a) on fait réagir les acides mono- et dicarboxyliques contenus dans le mélange d'acides dicarboxyliques aqueux avec un alcool de faible masse moléculaire pour obtenir les esters des acides carboxyliques correspondants,
b) on libère, dans une première étape de distillation, le mélange d'estérification obtenu, de l'alcool en excès et des produits à bas point d'ébullition,
c) on réalise, dans une deuxième étape de distillation, à partir du produit de fond, une séparation en une fraction ester essentiellement exempte de 1,4-cyclohexanediols et une fraction contenant au moins la plus grande partie de cyclohexanediols,
d) on hydrogène, par voie catalytique, la fraction ester essentiellement exempte de 1,4-cyclohexanediols et
e) on obtient, dans une étape de rectification, de l'hexanediol-1,6 à partir du produit d'hydrogénation, de manière connue en soi,
**caractérisé en ce que** l'on utilise un mélange d'acides dicarboxyliques aqueux qui contient plus de 20 ppm de cobalt et plus de 40 ppm de phosphore sous forme de phosphate, et **en ce que** l'on fait passer ce mélange d'acides dicarboxyliques aqueux sur un échangeur de cations et, après l'estérification selon l'étape (a), sur un échangeur d'anions.

2. Procédé selon la revendication 1, **caractérisé en ce que** l'on réalise le pré-traitement avec des échangeurs d'ions à lit fixe.

3. Procédé selon la revendication 1, **caractérisé en ce que** l'on réalise le pré-traitement avec deux lits fixes fonctionnant en alternance, que l'on peut également disposer l'un après l'autre, jusqu'à ce que la teneur en cobalt de la sortie du premier lit soit supérieure à 90% de la concentration d'entrée, ce pré-traitement étant alors suivi de l'arrêt et de la régénération du premier lit, lequel prend ensuite en charge la fonction du lit placé après.

4. Procédé selon la revendication 1, **caractérisé en ce que** l'on réalise le pré-traitement avec, à chaque fois, deux lits échangeurs d'anions disposés l'un après l'autre, jusqu'à ce que la teneur en phosphate de la sortie du premier lit soit supérieure à 50% de la valeur d'entrée, ce pré-traitement étant alors suivi de l'arrêt et de la régénération du premier lit, lequel prend ensuite en charge la fonction du lit placé après.

5. Procédé selon la revendication 1, **caractérisé en ce qu'**on lave l'échangeur de cations, avant la régénération, sans produits, avec de l'eau, et **en ce que** l'on mélange l'eau de lavage à la solution d'acides dicarboxyliques de départ.

6. Procédé selon la revendication 1, **caractérisé en ce qu'**on lave l'échangeur d'anions, avant la régénération, avec du méthanol, et **en ce que** l'on recycle la solution de lavage dans l'estérification.
